Europäisches Patentamt

European Patent Office

Office européen des brevets

(11) Numéro de publication: **0 494 569 A1**

# DEMANDE DE BREVET EUROPEEN

(21) Numéro de dépôt: **91420009.2**

(22) Date de dépôt: **09.01.91**

(51) Int. Cl.5: **A61M 5/32**

(43) Date de publication de la demande:
**15.07.92 Bulletin 92/29**

(84) Etats contractants désignés:
**CH DE GB LI**

(71) Demandeur: **Capy, Gilbert**
**La Botte Jarnioux**
**F-69640 Denice(FR)**

(72) Inventeur: **Capy, Gilbert**
**La Botte Jarnioux**
**F-69640 Denice(FR)**

(54) **Boîte de stockage d'aiguilles intramusculaires ou équivalentes à introduction latérale.**

(57) La boîte de stockage des aiguilles intramusculaires ou équivalentes est fermée par un volet flexible (1) qui permet, par déformation, d'introduire l'aiguille (10), en position sur l'embout (13) de la seringue, à l'intérieur de la boîte par transfert latéral (29) par l'ouverture latérale (14), et de la détacher de cette dernière en tirant suivant la direction (30) grâce à une retenue sur laquelle vient, s'appuyer le manchon (15) de fixation de l'aiguille (10) sur l'embout (13).

EP 0 494 569 A1

L'invention concerne un conteneur pour aiguilles intramusculaires ou équivalentes usagées.

L'apparition de maladies nouvelles qu'on ne sait pas guérir et qui se transmettent notamment par le sang, l'utilisation de seringues par les drogués, ont conduit à rechercher tous les moyens d'ampêcher la réutilisation des seringues usagées. Les axes de recherche sont divers, notamment on a cherché à faire en sorte que la seringue soit détruite à la fin de l'injection, on a conçu des récipients susceptibles de recevoir la seringue complète, et que l'on détruit ensuite, on a conçu des récipients munis d'un dispositif qui récupère l'aiguille et permet an outre la destruction de l'embout de fixation de l'aiguille sur la seringue. Les produits issus de ces concepts sont, soit encombrants, soit coûteux, ce qui réduit leur créneau d'utilisation. Le brevet FR 2603872 propose une boîte, susceptible de recueillir les aiguilles usagées, constituée d'une ouverture fermée par un écran mobile commandée manuellement par la main qui tient la boîte tandis que l'autre main présente l'aiguille par son extrémité pointue, soit seule, soit encore fixée sur l'embout de la seringue. La commande d'ouverture est constituée d'un bouton sur lequel on appuie ce qui comprime un ressort et déplace un volet qui permet de dégager une ouverture. Pour refermer il suffit de relâcher la pression sur le bouton, ce qui ramène le volet à sa position initiale. Si l'on présente l'aiguille seule il suffit de relâcher la bouton lorsque l'aiguille est tombée à l'intérieur. Si l'on présente l'aiguille encore fixée sur l'embout de la seringue, on introduit l'aiguille jusqu'à ce que l'embout de fixation non recouvert par la manchon de fixation de l'aiguille, soit à hauteur du volet; on relâche la pression sur la bouton, et la volet vient prendre appui sur l'embout de la seringue et constituer une retenue sur laquelle vient s'appuyer le manchon de fixation de l'aiguille; lorsqu'on tire sur la seringue, l'aiguille se sépare de la seringue et reste dans la boîte, tandis que la volet revient à sa position initiale et maintient la boîte fermée. L'inconvénient d'un tel produit, est qu'il implique que l'on présente l'aiguille par son bout pointu vers l'orifice de la boîte, au voisinage de la main qui tient cette dernière, et que c'est un produit constitué de plusieurs pièces qui impliquent du temps de montage. Dans ce brevet sont présentés deux autres produits sous les références PCT WO 82/00412 et E.P. 0.123.247. Le premier a une ouverture de la taille de l'embout de la seringue bordé de lamelles flexibles; lorsqu'on introduit l'extrémité pointue de l'aiguille dans cet orifice, les lamelles flexibles s'escamotent au passage du manchon de fixation de l'aiguille et reviennent an place pour constituer un retenue qui permet de séparer l'aiguille de la seringue en tirant sur cette dernière; Un tel système présente l'inconvénient qu'il faut présenter l'aiguille par la pointe et d'avoir une ouverture permanente par laquelle une aiguille peut partiellement ressortir pointe en avant si l'on renverse la boîte et blesser le manipulateur. Le deuxième brevet concerne un récipient qui est muni d'un bouchon rotatif qui s'ouvre et se ferme à la main dégageant une ouverture comprenant une retenue en forme de "V" permettant de prendre appui pour désolidariser l'aiguille de la seringue. L'inconvénient d'un tel système, est qu'il faut présenter l'aiguille par son extrémité pointue, et fermer manuellement la récipient.

L'objet de l'invention est de combiner les avantages de tous ces concepts ajoutés à des innovations de manière à obtenir un produit performant et bon marché. En effet, comme c'est un produit concernant la prévention des accidents, il est impératif qu'il sait largement diffusé, et qu'en conséquence il ne soit pas d'un coût ou d'une mise en oeuvre dissuasifs, enfin qu'il n'induise pas d'autres risques.

La Fig.1 montre la boîte avec son volet flexible.

La Fig.2 montre un détail de la boîte avec une seringue an position pour introduire l'aiguille usagée dans la boîte.

La Fig.3 montre la demi-boîte conçue de telle façon que l'assemblage de ces deux demi-boîte permette d'obtenir la boîte prête à l'emploi.

La Fig.4 montre une variante de l'invention comportant une protection du volet flexible.

La Fig.10 représente la couvercle de la boîte selon l'invention, comportant des éclatés, sur lequel vient se fixer la volet flexible.

La Fig.11 représente le corps de la boîte selon l'invention.

La Fig.12 représente le volet flexible avec un éclaté permettant de voir partiellement son profil.

La Fig.13 représente une coupe longitudinale du volet flexible en place sur la boîte représentée en coupe partielle. On y a aussi représenté le cas de l'utilisation d'une tige ressort pour augmenter la pression de fermeture.

La Fig.14 représente la boîte selon l'invention une fois qu'elle est assemblée.

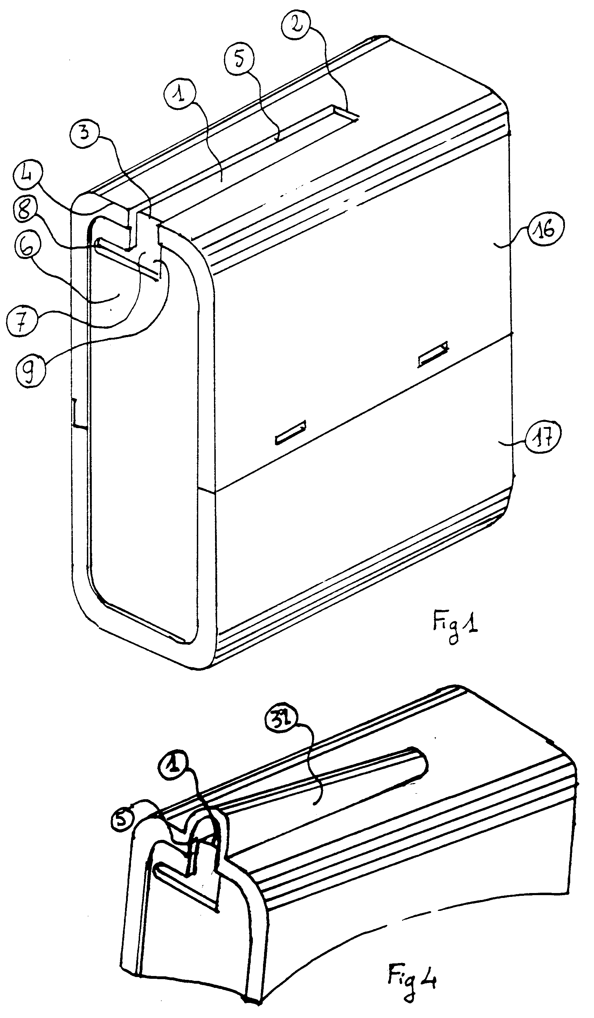

L'invention est constituée d'une boîte sensiblement parallèlépipédique Fig.1, comprenant un volet (1) de fermeture formé d'une lame de forme sensiblement rectangulaire ou trapézoidale, étroite et flexible d'une longueur sensiblement supérieure à celle des aiguilles utilisées et d'une largeur sensiblement supérieure à celle du manchon (15) Fig.2 de fixation des aiguilles. Une extrémité (2) Fig.1 du volet (1) est solidaire de la boîte, sa fixation étant située du côté interne de la boîte, tandis que l'autre extrémité (3) qui est libre, arrive à hauteur d'une arête (4) de la boîte. La flexion du volet se fait du côté intérieur de la boîte, le mouvement étant bloqué du côté extérieur. Seule une pression en provenance de l'extérieur peut provoquer un déplacement du volet. L'extrémité (2) peut-être fixée de multiples façons; on peut la fixer par emboîtage mécanique, par soudure, par collage, ou tout autre moyen connu. La boîte comporte une ouverture longitudinale (5) débouchant à hauteur de l'arête (4), au droit du volet de fermeture (1), de largeur légèrement intérieure à celle du volet (1). Au droit de l'ouverture longitudinale (5), sur l'autre face (6) de la boîte jouxtant l'arête (4) se trouve une découpe (7) de la paroi (6) qui part perpendiculairement à l'arête (4) avec une largeur sensiblement égale à celle de la découpe longitudinale (5), puis qui vire dans une direction sensiblement parallèle à l'arête et devient progressivement une encoche (8) plus étroite pour sa stabiliser à une largeur intérieure à celle du manchon (15) Fig.2 de fixation de l'aiguille (10) et légèrement supérieure à celle de l'embout (13) de la seringue (11). Une paroi (9) fixée sur le volet flexible (1), ferme côté intérieur, cette découpe (7). Sa surface est supérieure à celle de la découpe (7). Lorsqu'on appuie l'aiguille (10) Fig.2 fixée au bout de sa seringue (11) à plat sur le volet flexible (1) de manière, à ce que l'axe de l'aiguille (10) soit sensiblement dans l'axe du volet (1), et à ce que l'extrémité (12) du réservoir de la seringue (11) situé du côté de l'aiguille (10) soit pratiquement en contact avec l'arête (4), le volet (1) se déforme en s'enfonçant dans la boîte, et l'embout (13) de la seringue (11) s'engage dans la découpe (7) située sur la face perpendiculaire (6), qui se dégage par escamotage de la paroi de fermeture (9) Fig.1 qui est fixée sur le volet (1). La déformation du volet flexible (1) Fig.2 dégage, sur toute sa longueur, une ouverture latérale (14) communiquant avec l'intérieur de la boîte. Lorsqu'on fait parcourir, à l'embout (13) de la seringue(11), la découpe (7) suivant la direction (28), on maintient la seringue (11) sensiblement parallèle à elle-même. Lorsque la découpe (8) Fig.1 devient parallèle à l'arête (4), l'aiguille (10) Fig.2 qui se déplace suivant la direction (29) traverse alors l'ouverture (14) découverte par la déformation du volet (1) et se trouve à l'intérieur de la boîte, le rétrécissement de la découpe (8) constitue une retenue sur laquelle vient s'appuyer le manchon (15) de fixation de l'aiguille (10); lorsqu'on tire sur la seringue (11) suivant la direction (30), on sépare l'aiguille (10) de cette dernière, et l'aiguille (10) tombe dans la boîte. La paroi de fermeture (9) Fig.1 de la découpe (7) qui était en appui sur l'embout (13) Fig.2 de la seringue (11) est libérée ainsi que le volet flexible (1) qui reprend sa position initiale en refermant les ouvertures latérales (14). L'intérêt d'un tel système réside d'abord dans le fait que l'on présente l'aiguille (10) latéralement ce qui limite les risques de blessures provoquées par la présentation d'une aiguille par la pointe.

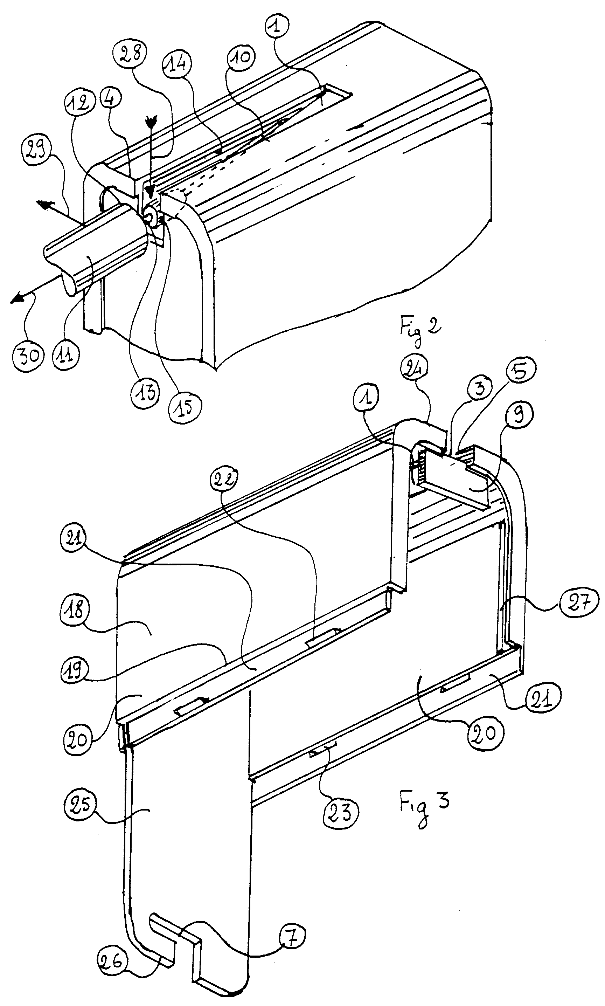

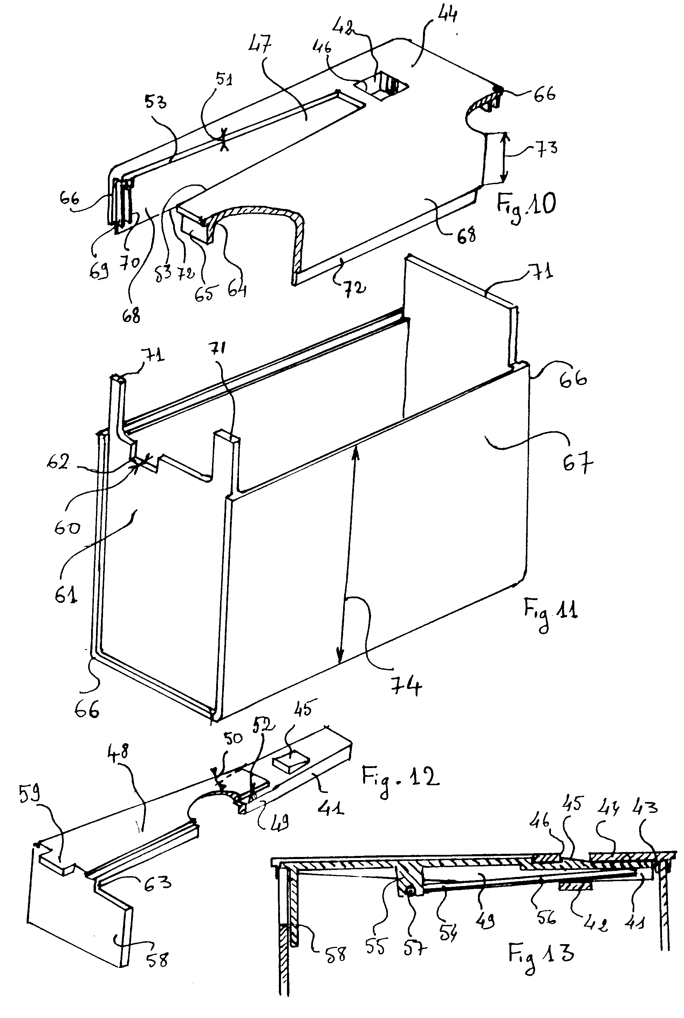

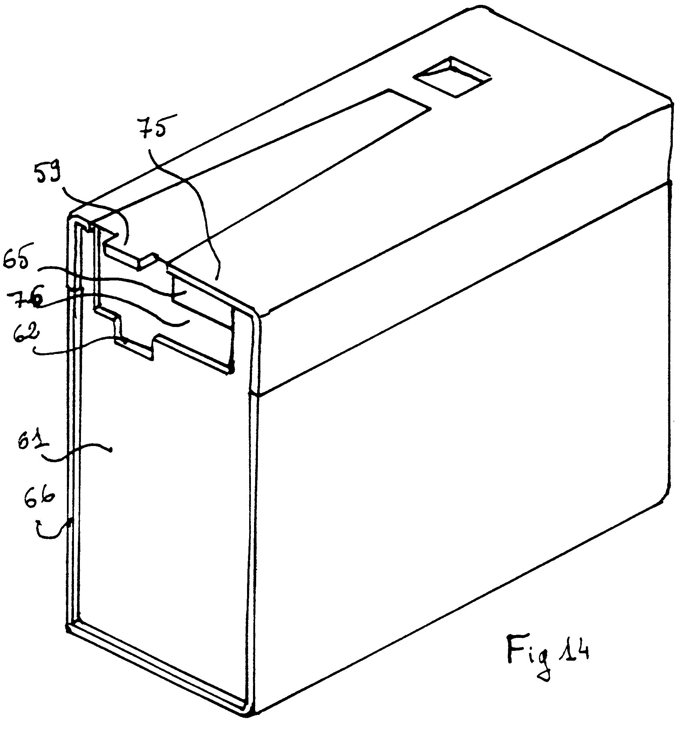

Ensuite la largeur de la fente permet aussi d'y introduire des bistouris jetables ainsi que les lames jetables de toutes sortes. On peut concevoir le produit pour que le volet flexible (1) soit fabriqué en même temps que la boîte, et à sa place définitive pour éviter toute reprise pour mettre en place le volet (1). La boîte est réalisée par injection de matière plastique en deux pièces (16) et (17) Fig.1 dont une comporte le volet flexible (1). On peut aussi concevoir la pièce (16) comportant le volet flexible (1) pour que l'assemblage de deux de cas pièces (16) constitue une boîte comportant deux volets flexibles (1). L'intérêt d'une telle opération est d'une part de limiter les investissements en outillage, et les stocks en produit fini puisqu'il n'y a plus qu'une seule pièce (18) Fig.3 à fabriquer et d'autre part d'augmenter la capacité pratique de la boite. Une façon de réaliser cette pièce (18) en matière plastique injectée consiste, par exemple, à fabriquer une demi boîte avec un plan de joint (19) passant sensiblement par le milieu de deux faces opposées (20). Ce plan de joint (19) comporte des zones de recouvrement (21) ainsi que des dispositifs de fixation par encliquetage (22) et (23). La face (24) de la boîte qui est sensiblement parallèle au plan de joint (19) comporte le volet flexible (1) avec la paroi (9) de fermeture de la découpe (7) Fig.1, qui lui est perpendiculaire. La face (6) de la boîte comportant la découpe (7) correspondant au volet flexible (1) ne fait pas partie de cette pièce (18) Fig.3. En contre partie, la face (25) opposée à celle correspondant à l'extrémité libre (3) du volet (1) est intégrée à cette pièce (18). Elle comporte à son extrémité (26) la découpe (7) correspondant au volet flexible (1) de la pièce (18) identique qui viendra s'y fixer. Elle sert de guidage aux deux pièces (18) pour permettre leur encliquetage au voisinage du plan de joint (19). Les plans de joints entre les pièces (18) comportent des chicanes (27) pour que las aiguilles ne puissent s'infiltrer entre les parois. Le volet flexible (1) est parallèle au côté interne de la face (24) de la boîte, et il est plus large que l'ouverture (5) pratiquée dans la paroi (24). Cette dernière peut comporter en outre des départs de cloison de faible hauteur au voisinage du volet flexible (1) pour empêcher les aiguilles de se positionner dans le plan de jonction du volet (1) et de la face interne de la paroi (24). Cette façon de procéder permet d'obtenir un moule simple et robuste. Il suffit alors d'une seule empreinte de la pièce (18) pour démarrer une production industrielle, et de livrer les pièces empilées les unes dans les autres afin de limiter la volume de transport. L'utilisateur prend deux pièces (18) pour las emboîter et constituer une boîte prête à l'emploi. Dans une variante de l'invention, on protège le valet flexible (1) Fig.4 par une paroi (31), solidaire des bords de l'ouverture longitudinale (5) laissant une ouverture suffisante pour pouvoir y introduire l'aiguille et son manchon de fixation, ainsi que des lames de bistouri ou des bistouris jetables. Cette paroi (31) dont la forme peut être variable, a pour but de protéger le volet flexible (1) contre des manoeuvres intempestives en cours de transport notamment, lorsque la boîte est transportée dans une sacoche mélangée à d'autres articles.

Pour que le volet flexible Fig.12 puisse présenter des caractéristiques d'élasticité convenables, il est réalisé dans un matériau plastique différent de celui de la boîte, et il est fixé mécaniquement à cette dernière; ce qui permet de choisir le matériau le mieux adapté aux conditions d'exploitation; dans une version préférée de l'invention le matériau choisi est une matière plastique à base de résine acétale qui présente l'avantage de posséder une limite élastique importante ainsi qu'une grande ténacité. Ce volet flexible est terminé par un manchon (41) qui s'enfile dans un étrier (42) Fig.10 et Fig.13 solidaire de la boîte et qui vient se bloquer en bout de course sur une butée (43) Fig.13 qui sert à le positionner; la paroi (44) Fig.10 et Fig.13 de la boîte au droit de l'étrier (42) étant supprimée, un cran de retenue (45) Fig.12 et Fig.13, solidaire de le volet flexible vient s'y loger et se mettre en butée contre le bord (46) Fig.13 de l'ouverture faisant face à l'étrier (42) pour empêcher le volet flexible, lorsqu'il est appuyé contre la butée (43), de revenir; le manchon (41) Fig.12 et Fig.13 est ainsi maintenu en place et permet une flexion élastique du volet flexible. Ce volet flexible Fig.12 est, dans une version préférée de l'invention, de section de surface sensiblement constante sur toute sa longueur, mais pour lui donner un moment d'inertie progressif entre son extrémité et son point de fixation, afin que la déformation soit régulièrement répartie, on lui donna une section en forme de "U" dont la partie centrale (48) sert à assurer la fermeture de l'ouverture (47) Fig.10 de la boîte, et les branches latérales (49) Fig.12 à faire varier le moment d'inertie du volet flexible; ainsi les branches latérales (49) sont plus hautes du côté du manchon de fixation (41) et la partie centrale (48) plus étroite; lorsqu'on s'éloigne du manchon de fixation (41) la hauteur des branches latérales (49) diminue tandis que la partie centrale (48) du volet flexible s'élargit; afin que le volet flexible puisse plaquer avec un effort minimum sur la paroi (44) Fig.10 de la boîte, la partie centrale (48) forme avec le manchon (41) un angle (50) de quelques degrés de manière à ce que, lorsque le manchon (41) est en place dans son étrier (42), la volet flexible, lorsqu'il vient fermer l'ouverture (47) Fig.10 en s'appuyant sur la paroi (44) subisse déjà une légère déformation élastique qui le plaque contre la paroi (44) de la boîte; comme le volet flexible peut subir en cours d'injection une légère déformation permanente dûe au retrait de la matière et qui le courbe vers l'intérieur de la boîte, la partie centrale (48) Fig.12 comporte un réhaussement de hauteur (52) sensiblement égale à l'épaisseur (51) Fig.10 de la paroi (44) de la boîte, de manière à ce que la partie centrale (48) Fig.12 du volet flexible puisse s'engager entre les deux bords (53) Fig.10 de l'ouverture (47) de la boîte et compenser les déformations du volet flexible pour éviter qu'elles ne se traduisant par une fente par laquelle les aiguilles pourraient passer. Si on veut augmenter la force d'appui du volet flexible sur la paroi (44) de la boîte, il est possible de positionner à l'intérieur du "U" une tige d'acier (54) Fig.13 qui prend appui sur l'étrier (42) de maintien du volet flexible, et sur le volet flexible par l'intermédiaire d'un support (55) solidaire de ce dernier; on peut donner par exemple à cette tige (54) la forme d'une épingle à cheveux dont les deux branches (56) s'appuient sur l'étrier (42) et la partie de liaison (57) des deux branches sur un support (55) injecté en même temps que le volet flexible; il n'est pas alors indispensable de donner au volet flexible une forme comme celle décrite précédemment pour assurer la forte d'appui sur la paroi (44) sans l'assistance d'un ressort. Il existe de multiples autres façons de réaliser les fonctions que nous venons de décrire, et qui font partie du domaine de l'invention mais celle que nous avons décrite nous paraât la plus économique. Pour limiter le débattement du volet flexible, on le prolonge, au delà de la paroi (58) perpendiculaire au volet flexible Fig.12, par une butée (59) d'une longueur correspondant sensiblement à l'épaisseur (60) Fig.11 de la paroi (61) de la boîte; lorsque le volet flexible atteint la déformation maximum nécessaire à son fonctionnement normal, la butée (59) Fig.14 vient s'appuyer sur la paroi (61) de la boîte délimitant l'encoche (76) permettant le passage du manchon de l'aiguille fixée à l'extrémité de la seringue; la zone d'appui (62) de la butée (59) du volet flexible forme un décrochement permettant un escamotage suffisant du volet flexible pour ne pas gêner le passage de l'extrémité de la seringue dans l'encoche (76). La paroi (58), perpendiculaire au volet flexible Fig.12, comporte un décrochement (63) qui, lorsque l'aiguille est en position dans l'encoche (76) Fig.14, permet au volet flexible de remonter légèrement de manière à empêcher le mouvement inverse de l'aiguille et de sa seringue; cela permet d'éviter que l'aiguille puisse ressortir intempestivement de la boîte en fonction de la façon dont on procède pour tirer sur la seringue.

Comme nous l'avons dit la recherche d'une déformation minimum du volet flexible, ainsi que le souci d'augmenter au maximum le volume utile de stockage de la boîte, conduit à positionner l'encoche (76) Fig.14 de retenue du manchon de l'aiguille la plus près possible du sommet (75) de la boîte; pour éviter que l'aiguille ne se coince entre la paroi supérieure (44) Fig.10 et la paroi (58), perpendiculaire au volet flexible Fig.12, on place au droit de la partie supérieure (65) Fig.14 de l'encoche (76) des rampes (64) Fig.10 qui font basculer l'aiguille vers l'intérieur de la boîte sous la poussée de la paroi (58), perpendiculaire au volet flexible Fig.12, qui remonte; pour réaliser ces rampes (64) Fig.10 par injection, une façon de les réaliser est de rendre solidaire la paroi supérieure (65) de l'encoche (76) Fig.14 munie de ses rampes (64) Fig.10, de la paroi supérieure (44) de la boîte tandis que la reste de l'encoche fait partie de la paroi (61) Fig.11 qui lui est perpendiculaire; cette façon de procéder permet notamment de renforcer la résistance mécanique de la partie supérieure (65) Fig.14 de l'encoche (76) et de simplifier de manière significative la construction des moules d'injection; en effet lorsque la totalité de l'encoche fait partie de la surface (61) Fig.11 la partie supérieure de l'encoche comporte une contre dépouille qui oblige à faire un moule comportant des chariots et elle reste extrêment fragile tandis que la fabrication et l'ajustage des rampes reste difficile.

Afin de parmettre de fixer éventuellement la boîte sur un support on aménage aux extrémités de la boîte et si possible sur tout la pourtour des retenues (66) Fig.14 dans lesquelles viendront sa loger les cliquets d'un support de fixation; lorsque la retenue (66) fait tout le pourtour de la boîte, elle permet de disposer d'un large éventail de possibilités de fixation et de positionnement de la boîte suivant que le support est notamment vertical ou horizontal. La boîte est contituée de deux parties contituées d'un couvercle Fig.10 et et d'un corps Fig.11 qui s'assemblent suivant des parois (67) et (68) qui se positionnent bout à bout ou des parois (44) ou (68) et (61) qui se positionnent perpendiculairement; dans ce dernier cas, dans une version préférée de l'invention, la paroi (61) qui s'appuie en bout sur la paroi (44) ou (68) qui lui est perpendiculaire, vient s'encastrer entre deux nervures (69) et (70) solidaires des paroi (44) et (68) empêchant tout débattement latéral; cette façon de procéder permet d'éviter d'avoir à encoller les zones de contact des deux parties de boîte sur toute leur longueur et ainsi de se limiter aux zones faciles (71) et (32) d'accès notamment par trempage dans le bac de colle ou de solvant; elle permet aussi d'assurer une fermeture aussi parfaite que possible de la boîte empêchant non seulement les aiguilles de passer mais les écoulements éventuels de liquide provenant des aiguilles; pour limiter les risques d'écoulement de liquide la boite est constituée d'un corps monobloc Fig.11 constituant un bac de rétention des aiguilles et d'un couvercle Fig.10 sur lequel est fixée la volet flexible Fig.12; la hauteur (73) Fig.10 de ce couvercle est uniquement fonction de l'esthétique et des contraintes techniques limitant la profondeur (74) Fig.11 du corps de la boîte.

## Revendications

1. Boîte pour le stockage d'aiguilles intramusculaires ou équivalentes usagées caractérisée en ce qu'elle comporte un volet flexible (1) fixé à l'extrémité (2) par emboîtage mécanique, et ou soudure, et ou collage, ou directement injecté en même temps que la boîte, fermant une ouverture (5), sur lequel le manchon (15) de l'aiguille (10), solidaire de la seringue (11), vient s'appuyer pour le déformer et dégager d'une part une ouverture latérale (14), permettant d'introduire l'aiguille (10) par translation par rapport à elle-même à l'intérieur de la boîte, d'autre part une découpe (7), située sur la face (6) qui est dans un plan perpendiculaire à l'ouverture (5), qui est fermée par une paroi (9), fixée perpendiculairement sur le volet flexible (1), et qui comporte une retenue (8) du manchon (15) de fixation de l'aiguille (10), permettant de la détacher de l'embout (13) de la seringue (11) par une simple traction, permettant ainsi, à l'aiguille (10) de tomber dans la boîte, et, en dégageant l'ouverture (8), au volet (1) de refermer la boîte en revenant élastiquement à sa position initiale, la volet (1) et la paroi (9) ayant une surface supérieure à celle des ouvertures (5) et (7) de manière à ne laisser aucune possibilité de passage dans une direction perpendiculaire aux parais concernées.

2. Boîte pour le stockage d'aiguilles intramusculaires ou équivalentes usagées suivant l'une quelconque des revendications précédentes caractérisée en ce que l'assemblage par clipsage de deux demi-boîtes identiques, constituées d'une seule pièce par injection directe du volet (1) et de la paroi (9) en même temps que la demi-boîte , permet d'obtenir une boîte complète prête à l'usage.

3. Boîte pour le stockage d'aiguilles intramusculaires ou équivalentes usagées suivant l'une quelconque des revendications précédentes caractérisée en ce que qu'une paroi (31) solidaire des bords de l'ouverture longitudinale (5) protège le volet (1) contre des manoeuvres intempestives.

4. Boîte de stockage d'aiguilles intramusculaires ou équivalentes usagées suivant l'une quelconque des revendications 1, 2, et 4, caractérisé en ce que le volet flexible Fig.12 est constitué d'une matière différente de celle de la boîte à laquelle elle est assemblée mécaniquement.

5. Boîte de stockage d'aiguilles intramusculaires ou équivalentes usagées suivant l'une quelconque des revendications précédentes, caractérisée en ce que le profil du volet comporte un moment d'inertie variable de manière continue depuis le manchon de fixation (41) jusqu'à son extrémité et que la partie flexible fait un angle (50) de quelques degrés permettant de la faire plaquer sur l'ouverture (47).

6. Boîte de stockage d'aiguilles intramusculaires au équivalentes usagées suivant l'une quelconque des revendications précédentes, caractérisée en ce qu'un décrochement (63) permet au volet flexible de remonter légèrement afin d'interdire le retour de l'aiguille et de la seringue assemblées.

7. Boîte de stockage d'aiguilles intramusculaires au équivalentes usagées suivant l'une quelconque des revendications précédentes, caractérisée en ce que la partie supérieure (65) de l'encoche (76) est solidaire du couvercle de la boîte Fig.10 et qu'elle comporte des rampes (64) évitant le coincament de l'aiguille lors de sa séparation de la seringue.

8. Boîte de stockage d'aiguilles intramusculaires ou équivalentes usagées suivant l'une quelconque des revendications précédentes caractérisé en ce que la paroi (68) s'ajuste avec la paroi (61) qui lui est perpendiculaire par l'intermédiaire de deux nervures (69) et (70) qui maintiennent en place la paroi (61).

9. Boîte de stockage d'aiguilles intramusculaires ou équivalentes usagées suivant, l'une quelconque des revendications précédentes, caractérisée en ce que les extrémités de la boîte comportant de retenues (66) permettant de la fixer sur un support par encliquetage.

10. Boîte de stockage d'aiguilles intramusculaires ou équivalentes usagées suivant l'une quelconque des revendications précédentes, caractérisée en ce qu'un ressort (54) formé d'une tige d'acier assure ou renforce la pression du volet flexible sur l'ouverture (47).

Fig 1

Fig 4

Fig 2

Fig 3

Fig. 10

Fig. 11

Fig. 12

Fig. 13

Fig 14

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | DE-A-3 118 749 (ARZNEIMITTEL APOTHEKER VETTER)<br>* page 4, ligne 24 - ligne 33 *<br>* page 5, ligne 1 - ligne 4 *<br>* page 6, ligne 21 - ligne 28 *<br>* page 7, ligne 9 - ligne 12; figures 1,13 *<br>--- | 1-2,5 | A61M5/32 |
| A | US-A-4 614 035 (W.M.ANDREWS)<br>* colonne 2, ligne 30 - ligne 60 *<br>* colonne 5, ligne 6 - ligne 23; figures 7-11,28 *<br>--- | 1-2,8 | |
| A | DE-A-4 013 013 (J.M.FARRE PONS)<br><br>* colonne 2, ligne 27 - ligne 55; figures 1,9 *<br>--- | 1,3-4,7,9 | |
| A | WO-A-8 800 477 (A.A.QUAYLE ET AL.)<br>* page 2, ligne 23 - page 3, ligne 5 *<br>* page 9, ligne 9 - page 10, ligne 12 *<br>* page 11, ligne 19 - ligne 22 *<br>* page 14, ligne 22 - ligne 27 *<br>* figures 15a - 15d, 26 *<br>--- | 1 | |
| E | FR-A-2 652 799 (G.CAPY)<br>* le document en entier *<br>----- | 1-3 | DOMAINES TECHNIQUES RECHERCHES (Int. Cl.5)<br><br>A61M<br>A61B |

Le présent rapport a été établi pour toutes les revendications

| Lieu de la recherche | Date d'achèvement de la recherche | Examinateur |
|---|---|---|
| LA HAYE | 04 SEPTEMBRE 1991 | NICE P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
 
& : membre de la même famille, document correspondant

EPO FORM 1503 03.82 (P0402)